(19) Europäisches Patentamt
European Patent Office
Office européen des brevets

(11) Numéro de publication: **0 281 477 B1**

(12) **FASCICULE DE BREVET EUROPEEN**

(45) Date de publication de fascicule du brevet: **22.07.92**

(51) Int. Cl.$^5$: **G01N 33/569**, G01N 33/543, G01N 33/537

(21) Numéro de dépôt: **88400487.0**

(22) Date de dépôt: **02.03.88**

(54) **Procédé de détection et de dénombrement d'antigènes particulaires dans un échantillon liquide, notamment des spores de clostridium tyrobutyricum dans le lait.**

(30) Priorité: **03.03.87 FR 8702862**

(43) Date de publication de la demande:
**07.09.88 Bulletin 88/36**

(45) Mention de la délivrance du brevet:
**22.07.92 Bulletin 92/30**

(84) Etats contractants désignés:
**AT BE CH DE ES GB GR IT LI LU NL SE**

(56) Documents cités:
**EP-A- 0 100 619**
**EP-A- 0 124 050**
**EP-A- 0 162 533**
**FR-A- 2 273 280**
**US-A- 4 562 147**

**BIOLOGICAL ABSTRACTS, vol.79, no.6, 1985, Philadelphia, PA (US); C.M.BOURGEOIS et al., p.364, no.48994**

(73) Titulaire: **BIOSYS S.A.**
**21 Ouai du Clos des Roses**
**F-60200 Compiegne(FR)**

(72) Inventeur: **Favreau, Bertrand**
**5 Ecart d'Aiguisy Lachelle**
**F-60190 Estrees St Denis(FR)**

(74) Mandataire: **Gillard, Marie-Louise et al**
**Cabinet Beau de Loménie 55, Rue d'Amsterdam**
**F-75008 Paris(FR)**

**EP 0 281 477 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet un procédé de détection et de dénombrement d'antigènes particulaires dans un échantillon liquide. La précision dudit dénombrement et son seuil de sensibilité le rendent particulièrement intéressant.

Dans la présente demande, on entend par antigène particulaire, toute entité qui se présente sous forme de particule, qui est elle-même un antigène ou qui porte de tels antigènes. On citera à titre d'exemples les spores, les bactéries, les cellules, des billes de matière synthétique sur lesquelles sont fixés des antigènes...

La mise en oeuvre du procédé selon l'invention permet la détection et le dénombrement, avec une sensibilité remarquable, de telles entités. Ledit procédé est une technique immunoenzymatique visant à créer des taches représentatives de l'antigène. Ces taches sont visibles par des moyens simples : à l'oeil nu ou à l'aide d'une loupe binoculaire ou d'un microscope photonique de faible grossissement (x 100).

Le procédé selon l'invention s'est notamment révélé particulièrement intéressant pour la détection et le dénombrement de spores de Clostridium tyrobutyricum dans les laits. Il répond dans cette application à un besoin de l'industrie fromagère.

En effet, le problème de la contamination des laits par les spores de Clostridium tyrobutyricum a pris une importance grandissante depuis les années 70, années à partir desquelles l'alimentation des vaches laitières à partir d'ensilage a été pratiquée.

Une technique de détermination de cette contamination consiste à concentrer les spores de Clostridium tyrobutyricum sur une membrane et à procéder ensuite au comptage des colonnies après culture des spores sur un milieu de germination approprié (Biological Abstracts, vol. 79, no. 6, 1985, page 364, résumé no. 48994, Philadelphie, PA, U.S.).

Cette contamination est fortement dommageable pour l'industrie fromagère. L'utilisation de laits pollués ou contaminés par lesdites spores entraîne au cours du processus d'affinage des pâtes fromagères des phénomènes dits "de fermentation tardive" ou "gonflements butyriques" qui provoquent de graves défauts de texture pouvant aller jusqu'à l'éclatement des meules et la dévalorisation ou la perte des fromages correspondants.

Les moyens dont disposent actuellement les laitiers pour tenter de faire face à cette pollution des laits sont peu nombreux ou inefficaces. On citera :

- la bactofugation, centrifugation poussée du lait qui permet de séparer les bactéries des autres constituants du lait. Cette opération est très onéreuse et dénature quelque peu le lait ;
- l'emploi de nitrates, inhibiteurs du développement de Clostridium tyrobutyricum. Leur emploi est interdit par la législation ;
- la pasteurisation. Cette opération est sans effet car les spores de Clostridium tyrobutyricum sont thermorésistantes.

On a également pensé, pour résoudre le problème, au triage des laits, afin de n'utiliser pour la fabrication des fromages que des laits exempts de cette pollution bactériologique. Malheureusement, il n'existe encore sur le marché aucun test de détection rapide. Les méthodes actuelles demandent 7 à 8 jours et dans l'intervalle, le lait a déjà été utilisé. De plus, ces méthodes sont fastidieuses et peu sûres...

Des recherches ont donc été entreprises pour mettre au point des tests, à la fois rapides, simples et efficaces, qui permettraient de déterminer l'étendue de la contamination du lait, notamment des tests basés sur des principes immunochimiques.

La technique de type E.L.I.S.A. a été essayée (Enzyme Linked Immuno Sorbent Assay = E.L.I.S.A.). Cette technique est par exemple présentée dans Journal of Clinical Pathology, 1978 , (31), 507-580. Elle s'est révélée, dans ce cas particulier, insatisfaisante. Sa limite de sensibilité, aux environs de 10 000 spores/ml, l'écartait de toute utilisation dans un test.

Selon l'invention, il est proposé un procédé de détection et de dénombrement des spores de Clostridium tyrobutyricum, très sensible. Son seuil de sensibilité permet la détection de 30 spores par ml d'échantillon.

Ledit procédé selon l'invention n'est nullement limité à la détection et au dénombrement de cet antigène particulaire spécifique, comme cela va ressortir de manière évidente de ses caractéristiques, présentées ci-après.

Le procédé de détection et de dénombrement d'antigènes particulaires dans un échantillon liquide selon l'invention, consiste à :

a) coupler lesdits antigènes avec un ou plusieurs anticorps, dont l'un au moins est conjugué à un premier enzyme ;
b) déposer les complexes antigènes-anticorps ainsi formés sur un gel contenant le substrat du premier

enzyme et un deuxième enzyme dont le substrat est le produit de la première réaction enzymatique ; ledit gel contenant également un révélateur - par formation d'un précipité - de la deuxième réaction enzymatique :

c) observer et/ou dénombrer les taches apparues sur le gel.

L'étape a) du procédé selon l'invention (couplage selon les techniques classiques de simple ou multiples couches ou les systèmes biotine-avidine) peut-être réalisée avant ou après toute technique de concentration-séparation des antigènes particulaires de l'échantillon. On citera notamment, les techniques de filtration, de centrifugation, d'adsorption sur un support solide (par exemple sur des billes, magnétiques ou non, sur des gels ...) de gradient de densité, de séparation biphasique par affinité ...

Le procédé selon l'invention, comme indiqué ci-dessus est basé sur la révélation d'une réaction immunoenzymatique, révélation qui se manifeste sous la forme de taches sur le gel.

On notera que cette technique diffère de la technique d'immunodiffusion radiale en gel décrite dans le brevet FR 2 273 280.

Ladite réaction immunoenzymatique doit engendrer un signal visible, net. C'est pourquoi, on choisit selon l'invention, des révélateurs agissant par formation d'un précipité. Ledit précipité doit être localisé autour de la particule. Cette localisation est dûe à la consistance du milieu réactionnel (gel). La diffusion des réactifs est ainsi limitée. De plus, le signal obtenu est visible, puisqu'il a été amplifié par diffusion-précipitation, par l' intervention du système bi-enzymatique.

La taille des taches de précipité est augmentée, elle peut même être multipliée par 100. Il est en effet parfois possible de coupler deux anticorps, dont l'un au moins est conjugué au premier enzyme ; le premier spécifique de la spore, le second spécifique du premier.

Les réactions immunoenzymatiques mises en jeu sont du type de celles, illustrées ci-après dans le cas où l'on utilise à l'étape a) du procédé selon l'invention, des anticorps couplés à la glucose oxydase et à l'étape b) un gel contenant du glucose, de la péroxydase et un révélateur de la péroxydase :

$$\text{D(+) glucose} \underset{}{\overset{\text{Glucose oxydase}}{\rightleftarrows}} \delta\text{-D-gluconolactone} + H_2O_2$$

$$\text{Révélateur} + H_2O_2 \xrightarrow{\text{Péroxydase}} H_2O + \text{précipité}$$

La révélation de la seconde réaction immunoenzymatique doit engendrer un précipité. On pourra donc utiliser comme révélateur toute substance compatible avec le milieu, qui engendre un précipité par oxydation et notamment une amine aromatique, telle l'aminoéthylcarbazole. Avantageusement le révélateur utilisé dans le procédé selon l'invention sera la diaminobenzidine. Par oxydation, elle génère un précipité brun.

Comme précisé ci-dessus, la révélation a lieu dans un gel pour engendrer un signal ponctuel, localisé sur les antigènes à observer, à dénombrer. La diffusion des produits de la réaction et des réactifs dissous doit être limitée. Elle doit toutefois pouvoir avoir lieu pour engendrer des taches.

Ledit gel peut être obtenu à partir de gélatineux type agar-agar, agarose, gélatine d'osséïne.

L'optimisation de la concentration des réactifs - péroxydase, substrat glucose oxydase : glucose, révélateur - dans ledit gel permet d'influer sur la taille du signal, qui consiste en une tache, une zone de coloration.

Avantageusement, le gel contient, outre les réactifs, un agent contrastant et/ou un anti-oxydant.

A titre d'agent contrastant, on peut utiliser le chlorure de nickel ($NiCl_2$). L'intervention d'un agent anti-oxydant en faible quantité est souhaitable. Elle permet la conservation du gel et évite que le révélateur ne s'oxyde de lui-même. Il n'interfère pas dans la réaction immunoenzymatique. On peut utiliser le bisulfite de sodium ($Na_2S_2O_5$).

Le dénombrement des antigènes est pratiqué par comptage des taches apparues sur le gel. Lesdites taches sont observées à l'oeil nu ou à l'aide d'une loupe binoculaire ou d'un microscope de faible grossissement (X 100).

Le procédé selon l'invention est avantageusement mis en oeuvre pour la détection et le dénombrement de spores de Clostridium tyrobutyricum dans le lait. Dans cette application particulière, on procède de la façon suivante :

a) on concentre par filtration les spores sur une membrane de composition chimique compatible avec les réactions immunoenzymatiques, intervenant à l'étape c) et qui retient lesdits antigènes ;
b) on couple lesdits antigènes avec un ou plusieurs anticorps, dont l'un au moins est conjugué à la glucose oxydase ;
c) on dépose les complexes antigènes-anticorps ainsi formés sur un gel contenant du glucose et de la péroxydase, en posant directement la face supérieure de la membrane sur le gel ; ledit gel contenant également un révélateur - par formation d'un précipité - de la seconde réaction enzymatique ;
d) on observe et/ou démontre les taches apparues sur le gel.

On concentre préalablement les antigènes sur une membrane par filtration.

La membrane utilisée doit évidemment avoir sa porosité adaptée à la taille des spores à filtrer.

On utilise généralement dans le cas des spores de Clostridium tyrobutyricum des membranes présentant un porosité de 0,45 μ.

De plus, ladite membrane doit avoir une composition chimique compatible avec les réactions immunoenzymatiques mises en jeu. Par exemple, on utilise des membranes en fluorure de polyvinylidène. A titre de révélateur de la seconde réaction enzymatique, on utilise avantageusement la diaminobenzidine.

Le précipité qui se forme dans le gel est coloré en brun.

Le gel utilisé est du type de ceux décrits ci-dessus. Il est préparé à partir d'une solution tampon et contient avantageusement pour 100 ml :

. 1000 mg d'agarose ;
. 50 mg de péroxydase ;
. 1500 mg de glucose ;
. 50 mg de diaminobenzidine ;
. 40 mg de $NiCl_2$ ;
. 10 mg de $Na_2S_2O_5$.

Le procédé selon l'invention peut être schématisé de la figure 1.

Selon l'invention, on a donc mis au point un procédé de dosage d'antigènes particulaires extrêmement sensible. Comme indiqué ci-dessus, il permet notamment le dosage des spores de Clostridium tyrobutyricum. La sensibilité atteinte de ce dosage est de 30 spores/ml.

L'invention sera mieux comprise à la lecture de l'exemple ci-après.

Un échantillon de lait contenant des spores de Clostridium tyrobutyricum est filtré sur une membrane de type Durapore hydrophile 0,45 μ (Millipore®).

Après cette filtration, la membrane est incubée dans l'appareil de filtration en présence d'une solution d'anticorps, préparés chez le lapin, dirigés contre l'antigène particulaire (dans le cas présent les spores) ; puis en présence d'un anticorps dirigé contre les immunoglobulines de lapin, couplé à la glucose oxydase.

Le filtre ainsi traité est déposé sur un gel contenant les éléments nécessaires au développement de la réaction enzymatique : glucose, péroxydase, diaminobenzidine.

On peut ainsi détecter jusqu'à $10^2$ particules par filtre.

## Revendications

**1.** Procédé de détection et de dénombrement d'antigènes particulaires dans un échantillon liquide, caractérisé en ce qu'il consiste à :
a) coupler lesdits antigènes avec un ou plusieurs anticorps, dont l'un au moins est conjugué à un premier enzyme ;
b) déposer les complexes antigènes-anticorps ainsi formés sur un gel contenant le substrat du premier enzyme et un deuxième enzyme dont le substrat est le produit de la première réaction enzymatique ; ledit gel contenant également un révélateur - par formation d'un précipité - de la deuxième réaction enzymatique ;
c) observer et/ou dénombrer les taches apparues sur le gel.

**2.** Procédé selon la revendication 1, caractérisé en ce que l'étape a) est réalisée avant ou après toute technique de concentration-séparation des antigènes particulaires de l'échantillon.

**3.** Procédé selon l'une des revendications 1 ou 2, caractérisé en ce que l'on utilise dans l'étape a) des anticorps conjugués à la glucose-oxydase ; et dans l'étape b) un gel contenant du glucose, de la péroxydase et un révélateur de la péroxydase.

**4.** Procédé selon la revendication 3, caractérisé en ce que ledit révélateur est une amine aromatique, tels

la diaminobenzidine ou l'aminoéthylcarbazole.

5. Procédé selon la revendication 1, pour la détection et le dénombrement de spores de Clostridium tyrobutyricum dans le lait, caractérisé en ce qu'il consiste à :
   a) concentrer par filtration les spores sur une membrane de composition chimique compatible avec les réactions immunoenzymatiques, intervenant à l'étape c) et qui retient lesdits antigènes ;
   b) coupler lesdits antigènes avec un ou plusieurs anticorps, dont l'un au moins est conjugué à la glucose oxydase ;
   c) déposer les complexes antigènes-anticorps ainsi formés sur un gel contenant du glucose et de la péroxydase, en posant directement la face supérieure de la membrane sur le gel ; ledit gel contenant également un révélateur - par formation d'un précipité - de la seconde réaction enzymatique ;
   d) observer et/ou démontrer les taches apparues sur le gel.

6. Procédé selon la revendication 5, caractérisé en ce que le révélateur est la diaminobenzidine.

7. Procédé selon l'une des revendications 5 ou 6, caractérisé en ce que le gel employé contient, pour 100 ml :
   . 1000 mg d'agarose ;
   . 50 mg de péroxydase ;
   . 1500 mg de glucose ;
   . 50 mg de diaminobenzidine ;
   . 40 mg de $NiCl_2$ ;
   . 10 mg de $Na_2S_2O_5$.

**Claims**

1. Method of detection and enumeration of particulate antigens in a liquid sample, characterised in that it consists in:
   a) coupling the said antigens with one or more antibodies, at least one of which is conjugated to a first enzyme;
   b) depositing the antigen-antibody complexes thus formed onto a gel containing the substrate of the first enzyme and a second enzyme whose substrate is the product of the first enzymatic reaction; the said gel also containing an indicator - by the formation of a precipitate - of the second enzymatic reaction;
   c) observing and/or enumerating the spots which appear on the gel.

2. Method according to Claim 1, characterised in that stage a) is carried out before or after any technique for concentrating-separating the particulate antigens in the sample.

3. Method according to one of Claims 1 or 2, characterised in that antibody-glucose oxidase conjugates are used in stage a); and a gel containing glucose, peroxidase and a peroxidase indicator are used in stage b).

4. Method according to Claim 3, characterised in that the said indicator is an aromatic amine such as diaminobenzidine or aminoethylcarbazole.

5. Method according to Claim 1, for the detection and the enumeration of Clostridium tyrobutyricum spores in milk, characterised in that it consists in:
   a) concentrating the spores, by filtration, on a membrane of chemical composition which is compatible with the immunoenzymatic reactions involved in stage c) and which retains the said antigens;
   b) coupling the said antigens with one or more antibodies, at least one of which is conjugated to glucose oxidase;
   c) depositing the antigen-antibody complexes thus formed onto a gel containing glucose and peroxidase, by directly placing the upper surface of the membrane onto the gel; the laid gel also containing an indicator - by the formation of a precipitate - of the second enzymatic reaction;
   d) observing and/or enumerating the spots which appear on the gel.

6. Method according to Claim 5, characterised in that the indicator is diaminobenzidine.

7. Method according to one of Claims 5 or 6, characterised in that the gel used contains per 100 ml:
   . 1000 mg of agarose;
   . 50 mg of peroxidase;
   . 1500 mg of glucose;
   . 50 mg of diaminobenzidine;
   . 40 mg of $NiCl_2$;
   . 10 mg of $Na_2S_2O_5$.

**Patentansprüche**

1. Verfahren zum Nachweis und zum Auszählen von teilchenförmigen Antigenen in einer flüssigen Probe, dadurch gekennzeichnet, daß es die folgenden Schritte umfaßt:
   a) Binden der Antigene an einen oder mehrere Antikörper, von denen mindestens einer mit einem ersten Enzym verbunden ist;
   b) Aufbringen der so gebildeten Antigen-Antikörperkomplexe auf ein Gel, das das Substrat des ersten Enzyms und ein zweites Enzym enthält, dessen Substrat das Produkt der ersten enzymatischen Reaktion ist, wobei das Gel daneben ein Entwicklungsreagens (durch die Bildung eines Präzipitats) der zweiten enzymatischen Reaktion enthält;
   c) Auffinden und/oder Auszählen der auf dem Gel erschienenen Plaques.

2. Verfahren nach Anspruch 1, dadurch gekennzeichnet, daß Schritt a) durchgeführt wird, bevor oder nachdem jede beliebige Technik der Aufkonzentrierung/Trennung der teilchenförmigen Antigene der Probe angewandt worden ist.

3. Verfahren nach einem der Ansprüche 1 oder 2, dadurch gekennzeichnet, daß man in Schritt a) mit Glucoseoxydase verbundene Antikörper und in Schritt b) ein Gel verwendet, das Glucose, Peroxydase und ein Peroxydaseentwicklungsreagens enthält.

4. Verfahren nach Anspruch 3, dadurch gekennzeichnet, daß das Entwicklungsreagens ein aromatisches Amin wie Diaminobenzidin oder Aminoethylcarbazol ist.

5. Verfahren nach Anspruch 1 zum Nachweis und zum Auszählen von Sporen von Clostridium tyrobutyricum in Milch, dadurch gekennzeichnet, daß es die folgenden Schritte umfaßt:
   a) Aufkonzentrieren der Sporen durch Filtration über einer Membran, deren chemische Zusammensetzung mit den immunoenzymatischen Reaktionen, die in Schritt c) ablaufen, verträglich ist und die die Antigene zurückhält;
   b) Binden der Antigene an einen oder mehrere Antikörper, von denen mindestens einer mit Glucoseoxydase verbunden ist;
   c) Aufbringen der so gebildeten Antigen-Antikörperkomplexe auf ein Glucose und Peroxydase enthaltendes Gel, das daneben ein Entwicklungsreagens der zweiten enzymatischen Reaktion (durch Bildung eines Präzipitats) enthält, indem die obere Oberfläche der Membran direkt auf das Gel gesetzt wird;
   d) Auffinden und/oder Auszählen der auf dem Gel erschienen Plaques.

6. Verfahren nach Anspruch 5, dadurch gekennzeichnet, daß das Entwicklungsreagens Diaminobenzidin ist.

7. Verfahren nach einem der Ansprüche 5 oder 6, dadurch gekennzeichnet, daß das verwendete Gel auf 100 ml enthält:
   - 1000 mg Agarose
   - 50 mg Peroxydase
   - 1500 mg Glucose
   - 50 mg Diaminobenzidin
   - 40 mg $NiCl_2$
   - 10 mg $Na_2S_2O_5$.

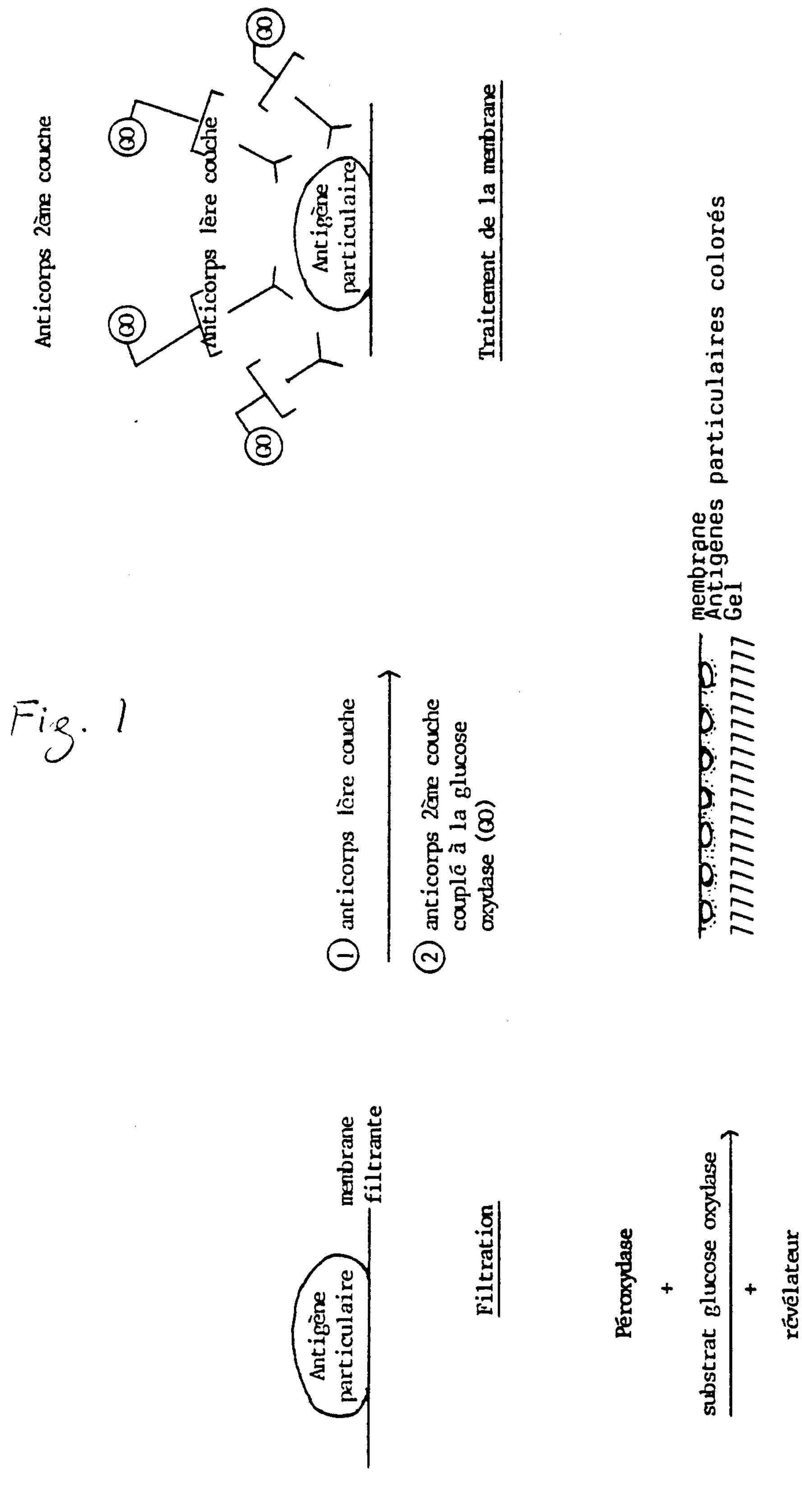
Antigène particulaire
membrane filtrante
Filtration
① anticorps 1ère couche
② anticorps 2ème couche couplé à la glucose oxydase (GO)
Anticorps 2ème couche
Anticorps 1ère couche
Antigène particulaire
GO
Traitement de la membrane
Péroxydase
+
substrat glucose oxydase
+
révélateur
membrane
Antigènes particulaires colorés
Gel

Fig. 1